# EUROPEAN PATENT APPLICATION

(11) **EP 4 810 468 A1**
(43) Date of publication of application: **23.09.2026**
(21) Application number: 25863879.0
(22) Date of filing: 29.07.2025
(51) Int. Cl.: C07C 29/76, C07C 29/04, C07C 31/10

(54) **METHOD FOR PREPARING ISOPROPYL ALCOHOL**

(30) Priority: 07.09.2024 KR 20240121957; 25.07.2025 KR 20250101553
(71) Applicant: LG Chem, Ltd., Seoul 07336 (KR)
(72) Inventor: OH, Jai June, Seoul 07796 (KR); KIM, Hyun Ju, Seoul 07796 (KR); LEE, Sung Kyu, Seoul 07796 (KR); RYU, Jin Sook, Seoul 07796 (KR); HWANG, Sung June, Seoul 07796 (KR); JANG, Kyung Soo, Seoul 07796 (KR)
(74) Representative: Goddar, Heinz J.
(86) International application number: PCT/KR2025/011231
(87) International publication number: WO 2026/054329

(57) **Abstract**

Provided is a method for preparing isopropyl alcohol including: supplying a propylene monomer and reaction water to a reactor as a feed stream to produce a gaseous reaction product including isopropyl alcohol, purifying isopropyl alcohol from the gaseous reaction product and recovering process water, and passing the purified isopropyl alcohol through an arsenic removal device including a first filtration layer, adsorbent layer, and a second filtration layer.

## Description

### [Technical Field]

### CROSS-REFERENCE TO RELATED APPLICATIONS

The present application claims the benefit of priorities to Korean Patent Application No. 10-2024-0121957, filed on September 7, 2024, and Korean Patent Application No. 10-2025-0101553, filed on July 25, 2025, the entire contents of which are incorporated herein as a part of the specification.

### Technical Field

The following disclosure relates to a method for preparing isopropyl alcohol, and more particularly, to a method for preparing high-quality isopropyl alcohol by removing arsenic included in isopropyl alcohol.

### [Background Art]

Isopropyl alcohol (IPA) is used for various purposes including a solvent for cleaning agents, raw materials for industrial paints and reagents, paints, inks, and the like in the electronics industry such as manufacture of semiconductor or liquid crystal display (LCD).

Isopropyl alcohol may be prepared by reacting a propylene monomer and water. For example, a propylene monomer and water are reacted in a gas phase in a reaction part to obtain a reaction product including isopropyl alcohol, an unreacted propylene monomer, unreacted water, and by-products, and the gaseous reaction product passes through a rear stage process tower to separate the propylene monomer, water, and the by-product, thereby purifying isopropyl alcohol.

In this process, water used as the reaction water is heated and then supplied to a reactor to produce isopropyl alcohol by a reaction with the propylene monomer, and unreacted water may be separated in an isopropyl alcohol purification part, cooled, recycled as process water to be used for washing an absorption tower and an organic material (hydrocarbons) removal tower, and partly discharged as wastewater. As such, water added during the process of preparing isopropyl alcohol is continuously circulated as process water, and the process water may include a small amount of arsenic.

However, since arsenic causes degradation of IPA quality, it needs to be removed from a final product. In general, a process of removing impurities by a method such as distillation is being performed in an IPA purification part, but since arsenic is decomposed or sublimated at or below an operation temperature of a distillation tower of the purification part of IPA, it is difficult to completely remove arsenic due to the phenomenon of vaporization into a gas phase by a distillation method. Thus, in order to prepare high-quality isopropyl alcohol, the development of technology for effectively removing arsenic in IPA is needed.

### [Disclosure]

### [Technical Problem]

An object of the present disclosure is to provide a method for obtaining high-quality isopropyl alcohol by continuously removing arsenic in purified isopropyl alcohol.

However, the object to be solved in the present disclosure is not limited to the object mentioned above, and other objects which are not mentioned may be clearly understood by a person skilled in the art from the following descriptions.

### [Technical Solution]

In one general aspect, a method for preparing isopropyl alcohol includes: supplying a propylene monomer and reaction water to a reactor as a feed stream to produce a gaseous reaction product including isopropyl alcohol; purifying isopropyl alcohol from the gaseous reaction product and recovering process water; and passing the purified isopropyl alcohol through an arsenic removal device including a first filtration layer, an adsorbent layer, and a second filtration layer.

In addition, the passing of purified isopropyl alcohol through an arsenic removal device including a first filtration layer, an adsorbent layer, and a second filtration layer may include passing the purified isopropyl alcohol through the first filtration layer, the adsorbent layer, and the second filtration layer inside the arsenic removal device sequentially.

### [Advantageous Effects]

The method for preparing isopropyl alcohol of the present disclosure may improve the quality of IPA by continuously removing arsenic in purified isopropyl alcohol by adsorption.

An effect which may be obtained in the present disclosure is not limited to the effects mentioned above, and other effects which are not mentioned will be understood clearly by a person with ordinary skill in the art to which the present disclosure pertains from the following description.

### [Description of Drawings]

FIG. 1 is a process diagram showing an entire process flow of the method for preparing isopropyl alcohol according to an exemplary embodiment of the present disclosure.
FIG. 2 is a process diagram which embodies the process A of FIG. 1
FIG. 3 shows a structure of an arsenic removal device used in the method for preparing isopropyl alcohol according to an exemplary embodiment of the present disclosure.
FIG. 4 shows a structure of an arsenic removal device used in the method for preparing isopropyl alcohol according to an exemplary embodiment of the present disclosure.
FIG. 5 shows a structure of an arsenic removal device used in the method for preparing isopropyl alcohol according to an exemplary embodiment of the present disclosure.
FIG. 6 is a graph showing an arsenic removal rate depending on an empty bed contact time in an exemplary embodiment of the present disclosure.
FIG. 7 is a graph comparing the number of days it took for an arsenic concentration in a product to reach 100% from 4,000% as compared with the management standard, depending on an arsenic removal rate in the product.

### [Best Mode]

The terms and words used in the description and claims of the present disclosure are not to be construed limitedly as having general or dictionary meanings but are to be construed as having meanings and concepts meeting the technical ideas of the present disclosure, based on a principle that the inventors are able to appropriately define the concepts of terms in order to describe their own disclosure in the best mode.

Regarding description of the drawings, similar reference numerals may be used for similar or related constituent elements.

A singular form of a noun corresponding to an item may include one or a plurality of items, unless otherwise explicitly stated in the relevant context.

In the present disclosure, each of the phrases such as "A or B", "at least one or A and B", "at least one of A or B", "A, B, or C", "at least one of A, B, and C", and "at least one of A, B, or C" may include any one of the items listed together with the corresponding phrase of the phrases or all possible combinations of them.

The term "and/or" includes a combination of a plurality of described related constituent elements or any one of a plurality of described related constituent elements.

The terms such as "1st" or "2nd", or "first" or "second" may be simply used for distinguishing a corresponding constituent element from other corresponding constituent elements, and the corresponding constituent elements are not limited in other aspects (e.g.: importance or order).

In addition, the terms such as "front surface", "back surface", "upper surface", "lower surface", "side surface", "left", "right", "upper", and "lower" used in the present disclosure are defined based on drawings, and the shape and position of each constituent element are not limited by the terms.

The terms such as "comprises" or "have" are intended to specify the presence of stated features, steps, operations, constituent elements, parts, or a combination thereof, but do not preclude the presence or addition of one or more other features, numerals, steps, operations, constituent elements, parts, or a combination thereof.

When it is described that a constituent element is "connected to", "combined with", "supported on", or "in contact with" other constituent elements, it includes not only the case in which the constituent elements are directly connected, combined, supported, or in contact, but also the case in which they are indirectly connected, combined, supported, or in contact through a third constituent element.

When it is described that a constituent element is positioned "on" other constituent element, it includes not only the case in which the constituent element is in contact with another constituent element, but also the case in which another constituent element is present between the two constituent elements.

In addition, when unique manufacture and material tolerances are suggested in the mentioned meaning, the terms such as "about" and "substantially" used in the present disclosure are used in the meaning of the numerical value or in the meaning close to the numerical value, and are used for preventing the disclosure mentioning a correct or absolute numerical value for better understanding of the present disclosure from being unfairly used by an unconscionable infringer.

The term "stream" used in the present disclosure may refer to a fluid flow in a process or may refer to a fluid itself flowing in a pipe. Specifically, the stream may refer to both a fluid itself flowing in a pipe connecting each device and a fluid flow. In addition, the fluid may include any one or more components of gas, liquid, and solid.

The term "upper portion" used in the present disclosure refers to, unless otherwise stated, a point at 0% to 20% of the height measured downward from the top of a device, and specifically, may refer to a top (tower top). In addition, the term "lower portion" refers to a point at 80% to 100% of the height measured downward from the top of a device, and specifically, may refer to a bottom (tower bottom).

In addition, "pressure" mentioned in the present disclosure refers to a gauge pressure measured based on atmospheric pressure.

The term "empty bed contact time (EBCT)" used in the present disclosure refers to a time during which a continuously flowing fluid substantially resides in a device or a specific area when passing the fluid through the device or the specific area and is calculated by "(volume of device) / (volume flow rate of fluid passing through device)" or "(volume of specific area) / (volume flow rate of fluid passing through specific area)", and the unit is hour (h). For example, when a fluid of 0.5 m³/h continuously flows into a device having a volume of 1 m³, the empty bed contact time is (1/0.5)=2h, which means that the fluid residing in the device for 2 hours.

When catalysts exchange in the process of preparing IPA, arsenic is circulated in the process for the reason of arsenic outflow in a carrier and the like, and thus, arsenic is eluted in the IPA product. Specifically, though IPA undergoes a process of being condensed into a gas phase and purified, a part of arsenic in the process water is decomposed and sublimated, and arsenic is also detected in a gaseous IPA stream. Therefore, since it is difficult to completely remove arsenic by a distillation method, circulation is performed for a long time in a state of containing arsenic in the process water, and arsenic is detected in a product, resulting in degradation of IPA quality.

Thus, in the present disclosure, high-quality isopropyl alcohol is intended to be prepared by continuously removing arsenic present in IPA in the process of preparing isopropyl by adsorption.

The method for preparing isopropyl alcohol according to an exemplary embodiment of the present disclosure includes: (S1) supplying a propylene monomer and reaction water to a reactor as a feed stream to produce a gaseous reaction product including isopropyl alcohol; (S2) purifying isopropyl alcohol from the gaseous reaction product and recovering process water; and (S3) passing the purified isopropyl alcohol through an arsenic removal device. In addition, the arsenic removal device may have a structure in which the first filtration layer, the adsorbent layer, and the second filtration layer are sequentially laminated inside.

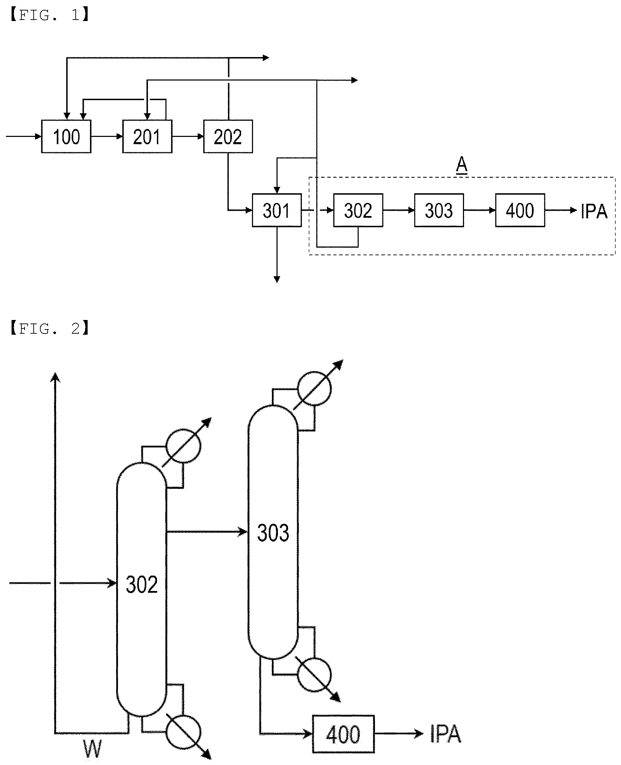

FIG. 1 illustrates the method for preparing isopropyl alcohol according to an exemplary embodiment of the present disclosure, in which isopropyl alcohol may be prepared using a system including a reactor 100, an absorption tower 201, a gas purification part 202, an organic material removal tower 301, a water removal tower 302, an IPA separation part 303, and an arsenic removal device 400.

According to an implementation example, a gaseous reaction product obtained in the reactor 100 passes through the absorption tower 201, the gas purification part 202, the organic material removal tower 301, the water removal tower 302, and the IPA separation part 303 to obtain purified IPA, and water recovered in the lower portion of the water removal tower is used as process water.

FIG. 2 shows the part A of FIG. 1 which is a process tower at the rear stage, more specifically. Referring to FIG. 2, purified IPA which has gone through a water removal tower 302 and an IPA separation part 303 is passed through the arsenic removal device 400 to remove arsenic before recovering a final product, thereby obtaining high-quality IPA.

The arsenic removal device 400 may be appropriately designed and changed depending on the flow rate of purified IPA, and for example, the empty bed contact time (EBCT) of the arsenic removal device may be designed to be 10 minutes or more, 30 minutes or more, or 1 hour or more and 3 hours or less, 4 hours or less, or 5 hours or less. When the empty bed contact time range is satisfied, arsenic removal efficiency may be improved. In addition, when the arsenic removal efficiency is improved, product production volume may be increased, thereby improving economic feasibility.

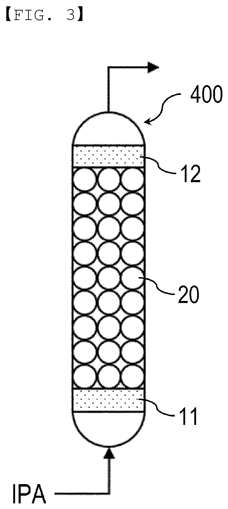

FIG. 3 shows the structure of the arsenic removal device 400 according to an exemplary embodiment. Referring to FIG. 3, the arsenic removal device 400 may have a structure in which the first filtration layer 11, the adsorbent layer 20, and the second filtration layer 12 are sequentially laminated.

In addition, in FIG. 3, the arsenic removal device is vertically shown, but the arsenic removal device may be placed in a horizontal direction, if necessary. Accordingly, the purified IPA may be added to one side of the arsenic removal device 400, pass through the first filtration layer 11, the adsorbent layer 20, and the second filtration layer 12 sequentially, and then be discharged to the other side.

The first filtration layer 11 and the second filtration layer 12 are provided in order to suppress elution of the arsenic adsorbent described later and impart a physical filtration effect, and the type may be the same or different. As the first filtration layer 11 and the second filtration layer 12, for example, activated carbon, sand, zeolite, silica gel, metal mesh net, or the like may be used.

In addition, the first filtration layer 11 and the second filtration layer 12 in the arsenic removal device 400 may be independently of each other provided to have the empty bed contact time of 1 to 30 minutes, specifically 1 to 20 minutes, and more specifically 1 to 10 minutes. The elution suppression of the adsorbent and the physical filtration effect may be maximized by satisfying the range.

As illustrated in FIG. 3, the adsorbent layer 20 is provided in order to remove a trace amount of arsenic included in the purified IPA by adsorption and is placed between the first filtration layer 11 and the second filtration layer 12. The arsenic adsorbent usable as the adsorbent layer 20 may be, for example, a metal oxide-based adsorbent such as iron hydroxide, activated alumina, titanium oxide, an ion exchange resin, α-iron oxide, and γ-iron oxide, and an ion exchange resin, and the like. The arsenic adsorbent may have different sizes depending on the shape and type. For example, the adsorbent based on the metal oxide may be in a pellet form or granular form, and the size may be in a range of 0.5 mm to 100 mm, but is not limited thereto. In addition, the ion exchange resin may be a gel or microporous type, and the size may be 300 µm to 1,200 µm, but is not limited thereto.

In addition, the adsorbent layer 20 may be provided for 10 minutes or more, 30 minutes or more, or 1 hour or more and 3 hours or less, 4 hours or less, or 5 hours or less in the arsenic removal device 400. Arsenic included in the purified IPA may be sufficiently removed by satisfying the range.

More specifically, an arsenic removal rate after passing through the arsenic removal device 400 may satisfy 20% or more, specifically 25 wt% to 95 wt%, and more specifically 30 wt% to 90 wt%, based on 100 wt% of the arsenic content included in the isopropyl alcohol before passing through the arsenic removal device 400.

In addition, the temperature of IPA passing through the arsenic removal device 400 may be 40°C to 150°C or 40°C to 60°C, and the temperature range in which arsenic may be removed may be different depending on the type of arsenic adsorbent.

According to an exemplary embodiment of the present disclosure, the adsorbent layer 20 may be divided into 1 to n areas, and the first to the nth areas may be filled with the first to nth adsorbent, respectively. Herein, each area may be filled with the same or different types of adsorbent, but includes at least one arsenic adsorbent. That is, the types of first to nth adsorbents may be the same as or different from each other. As compared with the structure in which the adsorbent layer is not divided, in the structure in which the adsorbent layer is divided into a plurality of areas, the length of a flow path through which the IPA passes the adsorbent layer is increased, and thus, an area and time in and during which the IPA comes into contact with the arsenic adsorbent are sufficiently secured. Accordingly, the arsenic adsorbent efficiency may be improved without changing the size of a device.

In addition, when the adsorbent layer 20 having a structure in which the inside of the arsenic removal device is divided into first to nth areas is provided, when the IPA passes through the arsenic removal device 400, the IPA may be supplied to one side of a first area 1, pass from the first area to the nth area sequentially, and then be discharged in one direction selected from one side and the other side of the nth area. For example, when the inside is divided into the odd number of areas, IPA may be discharged to the other side of the nth area, and when the inside is divided into the even number of areas, IPA may be discharged to one side of the nth area.

In addition, n may be an integer of 2 to 5, preferably 3 or 4. The inside of the arsenic removal device 400 may be divided into 2 or more, preferably 2 to 5, and more preferably 3 or 4 areas.

According to an exemplary embodiment, the first to nth areas may be divided by first to nth packings. The first to nth packings have a structure in which a plurality of packing members are radially arranged to have an overall cylindrical shape, and each packing member may be formed to have a sector-shaped or triangular sector-shaped cross section. Accordingly, the first to nth areas may be detached by a packing method, respectively, and thus, the adsorbent may be exchanged, if necessary. Herein, each of the first to nth packings may be filled with the same or different types of adsorbents.

In addition, a flow path in which at least one side is open may be provided between areas adjacent to each other among the first to nth areas so that IPA may move between the two areas. However, though the first area (that is, first packing) in which an IPA inlet is positioned and the nth area (that is, nth packing) in which an IPA outlet is positioned are adjacent to each other, there should be no open flow path therebetween. More specifically, a flow path open in the other side (C, E) direction is formed between an odd numbered packing (for example, the first packing) and an even numbered packing (for example, the second packing), and a flow path open in the other side (B, D) direction may be formed between an even numbered packing (for example, the second packing) and an odd numbered packing (for example, the third packing). Through the structure, IPA added to the adsorbent layer may come into contact with the first to nth adsorbents in the first to nth packing and then be discharged.

Meanwhile, in order to prevent the adsorbent from flowing out of the arsenic removal device, among the areas divided by the packing method, the first filtration layer 11 may be provided in the IPA inlet side of the first area (that is, first packing) through which the IPA first passes, and the second filtration layer 12 may be provided in the IPA outlet side of the nth area (that is, nth packing) through which IPA passes at the end, but is not limited thereto. The type, shape, size, and empty bed contact time of the adsorbent layer and the filtration layer are as described above.

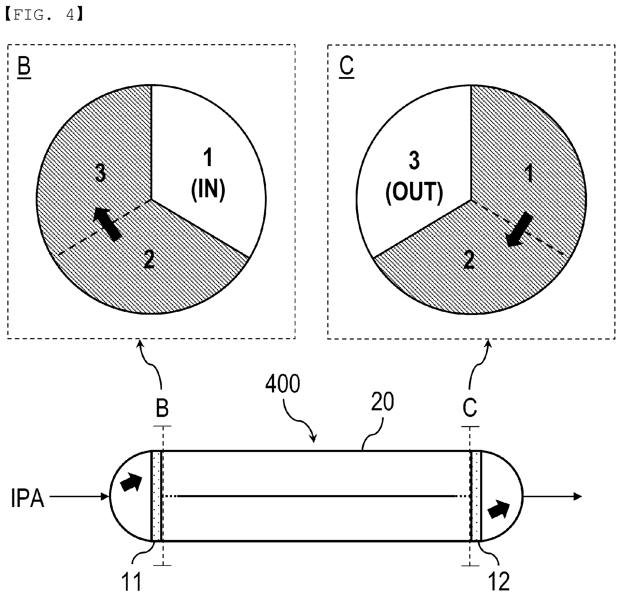

FIG. 4 shows a structure in which the first packing to the third packing 1, 2, 3 are provided as the adsorbent layer 20 in the arsenic removal device 400 according to an exemplary embodiment; and the lower drawing is a longitudinal sectional view of the device, the upper left drawing is a cross sectional view of one side (B) of the device, and the upper right drawing is a cross sectional view of the other side (C) of the device.

As shown in FIG. 4, in the case in which the number of inner areas divided by a packing method is an odd number, when IPA is added to one side of the arsenic removal device, IPA may pass through the adsorbent in each area, and then be discharged to the other side of the arsenic removal device, which is thus preferred.

Specifically, referring to FIG. 4, when the inside of the arsenic removal device 400 according to an exemplary embodiment is divided into a first packing 1, a second packing 2, and a third packing 3, the IPA may be added to one side of the first packing 1, pass through the second packing 2, and then be discharged to the other side of the third packing 3.

More specifically, the device may have a structure in which one side (B) of the surface (dotted line) where the second packing 2 and the third packing 3 meet is open, and the other side (C) of the surface (dotted line) where the first packing 1 and the second packing 2 meet is open. In addition, one side (B) of the first packing 1 may have a structure having a pipe connected or open so that IPA may be added thereto, and the other side (C) of the third packing 3 may have a structure having a pipe connected or open so that IPA may be discharged therefrom. The arsenic removal device 400 has the structure as such, thereby moving IPA supplied in the direction of one side (B) to "one side of the first packing 1 → the other side of the first packing 1 → the other side of the second packing 2 → one side of the second packing 2 → one side of the third packing 3 → the other side of the third packing 3" sequentially, and finally discharging IPA in the direction of the other side (C) of the arsenic removal device 400.

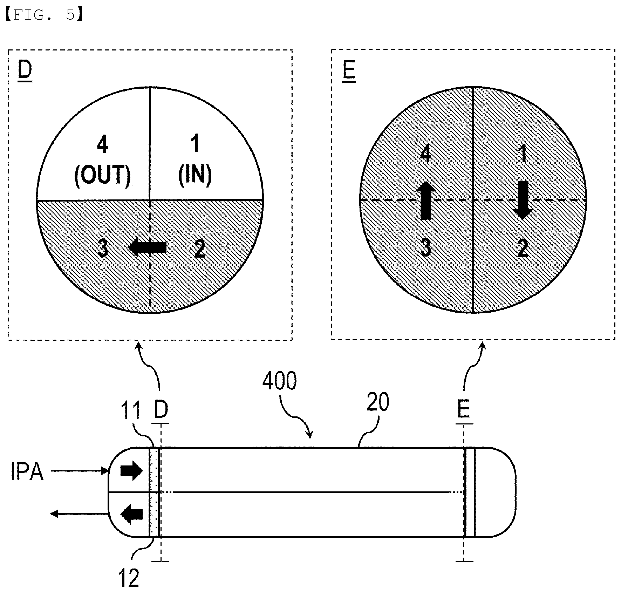

FIG. 5 shows a structure in which the first packing to the fourth packing 1, 2, 3, 4 are provided as the adsorbent layer 20 in the arsenic removal device 400 according to an exemplary embodiment; and the lower drawing is a longitudinal sectional view of the device, the upper left drawing is a cross sectional view of one side (D) of the device, and the upper right drawing is a cross sectional view of the other side (E) of the device.

As shown in FIG. 5, in the case in which the number of inner areas divided by a packing method is an even number, when IPA is added to one side of the arsenic removal device, IPA may pass through the adsorbent in each area, and then be discharged in the same direction of the IPA addition.

Specifically, referring to FIG. 5, when the inside of the arsenic removal device 400 according to an exemplary embodiment is divided into a first packing 1, a second packing 2, a third packing 3, and a fourth packing 4, the IPA may be added to one side (B) of the first packing 1, pass through the second packing 2 and the third packing 3, and then be discharged through the fourth packing 4.

More specifically, the device may have a structure in which one side (D) of the surface (dotted line) where the second packing 2 and the third packing 3 meet is open, and the other side (E) of the surface (dotted line) where the first packing 1 and the second packing 2 meet and the surface (dotted line) where the third packing 3 and the fourth packing 4 meet are open. In addition, one side (D) of the first packing 1 and the fourth packing 4 may independently of each other have a structure having a pipe connected or open so that IPA may be added or discharged. The arsenic removal device 400 has the structure as such, thereby moving IPA supplied in the direction of one side (D) to "one side of the first packing 1 → the other side of the first packing 1 → the other side of the second packing 2 → one side of the second packing 2 → one side of the third packing 3 → the other side of the third packing 3 → the other side of the fourth packing 4 → one side of the fourth packing 4" sequentially, and finally discharging IPA in the direction of one side (D) of the arsenic removal device 400.

Meanwhile, a part of the process water (W) may be transferred to an absorption tower 201 and an organic material removal tower 301, respectively and used as absorption water and washing water, and the rest may be discharged as wastewater.

Usually, water used as a raw material for preparing isopropyl alcohol, that is, reaction water is supplied to the reactor for a gaseous reaction with the propylene monomer.

Process water (W) may include washing water and unreacted water which is recovered from a process of purifying isopropyl alcohol by separating unreacted propylene monomer, by-products, and the like, while the gaseous reaction product passes through the absorption tower 201, the organic material removal tower 301, the water removal tower 302, and the like which are the rear stage processes. Referring to FIG. 1, process water (W) recovered from the water removal tower 302 is recycled for washing of the process tower at the front stage (for example, absorption tower 201 and organic material removal tower 301).

In addition, the process water (W) may pass through a heat exchanger (not shown) and be cooled. For example, the cooled process water may be branched and used as the washing water of the absorption tower 201 and the washing water of the organic material removal tower 301. In addition, a part of the cooled process water may be treated as wastewater, and, if necessary, pass through an additional cooler and then be branched, and a part may be used for adjusting the temperature of the washing water and the rest may be discharged as wastewater.

Reaction water is supplied to the reactor 100 and reacts with the propylene monomer in a gas phase to obtain a reaction product including isopropyl alcohol.

Since the propylene monomer used as a raw material is only partly used in the reaction, the reaction product may include 65 wt% to 85 wt% of the unreacted propylene monomer, 4 wt% to 8 wt% of isopropyl alcohol, and 5 wt% to 30 wt% of water. In addition, the reaction product may further include high boiling point organic materials such as isopropyl ether (DIPE) and hexene, and other by-products such as acetone and n-propyl alcohol (NPA). Therefore, a rear stage process for purifying isopropyl alcohol from the reaction product is performed.

First, the gaseous reaction product discharged from the reactor 100 is supplied to an absorption tower 201 and brought into contact with the washing water to obtain an aqueous solution including isopropyl alcohol. As described above, a part of the process water (W) recovered in the water removal tower 302 at the rear stage may be used as the washing water of the absorption tower 201.

Specifically, the reaction product may be supplied to the lower stage of the absorption tower 201, the washing water may be supplied from the upper stage of the absorption tower 201, gaseous isopropyl alcohol is absorbed by the washing water and obtained as a lower liquid stream, and a gaseous stream including the unreacted propylene monomer may be separated in the upper portion and recovered to the reactor 100.

The lower liquid stream of the absorption tower 201 may include the unreacted propylene monomer in a small amount, for example, 5 wt% or less or 2 wt% to 5 wt%, in addition to isopropyl alcohol and unreacted water.

According to an exemplary embodiment of the present disclosure, the volume flow rate of the washing water supplied to the absorption column 201 may be 15 vol% to 40 vol% or 15 vol% to 35 vol% of the flow rate of the reaction product. When the washing water is supplied at the flow rate within the range, an excessive increase in energy expense for recovering the washing water in the rear stage may be prevented, while simultaneously improving absorbency of isopropyl alcohol included in the reaction product.

The absorption tower 201 may be operated at a temperature of 90°C to 100°C or 90°C to 95°C and a pressure of 25 kg/cm² (g) to 40 kg/cm² (g) or 25 kg/cm² (g) to 35 kg/cm² (g). When the operation conditions are satisfied, an upper discharge stream including the unreacted propylene monomer and a lower discharge stream including isopropyl alcohol may be effectively separated.

The liquid stream including isopropyl alcohol separated from the absorption tower 201 may be supplied to a gas purification part 202 and separated into an upper stream including low-boiling point components and a lower stream including isopropyl alcohol, water, and by-products.

The upper stream including low-boiling point components separated in the gas purification part 202 may include an unreacted propylene monomer and inert gas (for example, ethane or propane).

The lower stream separated in the gas purification part 202 may include isopropyl alcohol, water, and by-products (for example, isopropyl ether (DIPE), hexene, n-propyl alcohol (NPA), and the like).

The gas purification part 202 may include one or more flash drums and one or more gas purification towers. First, low-boiling point components are first separated in the flash drum and are supplied to the gas purification tower again to separate the unreacted propylene monomer and the low-boiling point components of inert gas in the upper portion and a small amount of high-boiling point components in the lower portion.

The unreacted propylene monomer separated in the gas purification part 202 may be recovered to the reactor 100, and the inert gas may be branched for exhaust.

Meanwhile, the lower stream of the gas purification part 202 is transferred to an IPA purification part for recovering isopropyl alcohol. The IPA purification part includes an organic material removal tower 301, a water removal tower 302, and an IPA separation part 303.

First, the lower stream from the gas purification part 202 is supplied to the organic material removal tower 301 and brought into contact with washing water in the organic material removal tower 301 to be separated into a liquid phase including isopropyl alcohol and water and a liquid phase including an organic material. As described above, a part of the process water (W) recovered in the water removal tower 302 at the rear stage may be used as the washing water of the organic material removal tower 301.

An alcohol component is dissolved in the washing water, the liquid phase including isopropyl alcohol and water of the organic material removal tower 301 may be separated to the lower portion, and the remaining liquid phase including an organic material may be removed in a layer separator (decanter) at the rear stage of the connected condenser.

In an exemplary embodiment of the present disclosure, the volume flow rate of the washing water supplied to the organic material removal tower 301 may be 60 vol% to 100 vol% or 65 vol% to 95 vol% the flow rate of the lower stream of the gas purification part 202. When the washing water is supplied at the flow rate within the range, an excessive increase in energy expense for recovering the washing water in the rear stage may be prevented, while simultaneously improving absorbency of isopropyl alcohol included in the reaction product.

The liquid phase including isopropyl alcohol and water separated in the organic material removal tower 301 may include 3 wt% to 10 wt% of isopropyl alcohol and 90 wt% to 97 wt% of water. That is, since most of the liquid phase includes washing water, separation is needed.

Therefore, the liquid phase including isopropyl alcohol and water separated in the organic material removal tower 301 is supplied to the water removal tower 302 and separated into an upper stream including isopropyl alcohol and a lower stream including water.

The upper stream separated in the water removal tower 302 includes an azeotropic mixture including isopropyl alcohol and water, and for example, may include 80 wt% to 90 wt% of isopropyl alcohol and 10 wt% to 20 wt% of water.

Meanwhile, the lower stream from the water removal tower 302 may be recovered to process water (W), and a part may be recycled as washing water and/or absorption water and the rest may be discharged as wastewater.

The water removal tower 302 may be operated at a temperature of 70°C to 150°C or 80°C to 140°C and a pressure of 1 kg/cm² (g) to 5 kg/cm² (g) or 1 kg/cm² (g) to 2 kg/cm² (g). When the operation conditions are satisfied, the azeotropic mixture of isopropyl alcohol and water may be effectively separated.

Thereafter, an azeotropic mixture stream separated in the water removal tower 302 is supplied to an IPA separation part 303 including an IPA purification tower and a solvent recovery tower and the arsenic removal device 400 to recover IPA.

For example, when an organic solvent (for example, cyclohexane, benzene, and the like) is added to the IPA purification tower of the IPA separation part 303 as an azeotropic agent, azeotropy of isopropyl alcohol and water may be damaged to obtain highly purified isopropyl alcohol in the lower portion. Herein, the lower discharge stream from the IPA purification tower including purified IPA may pass through the arsenic removal device 400 as described above to remove arsenic. In addition, a stream including an azeotropic agent and water may be separated to the upper portion of the IPA purification tower and then supplied to a solvent recovery tower to separate the upper stream of the azeotropic agent and the lower stream of water, and the upper stream of the azeotropic agent may be refluxed to the IPA purification tower.

In the present disclosure, if necessary, devices such as a distillation column, a condenser, a reboiler, a valve, a pump, a separator, and a mixer may be further used.

Hereinabove, the method for preparing isopropyl alcohol according to the present disclosure has been described and illustrated in the drawings, but the description and the illustration in the drawings are the description and the illustration of only core constitutions for understanding of the present disclosure, and in addition to the process and apparatus described above and illustrated in the drawings, the process and the apparatus which are not described and illustrated separately may be appropriately applied and used for carrying out the method for preparing isopropyl alcohol according to the present disclosure.

Hereinafter, the present disclosure will be described in detail through the following examples. However, the following examples are intended to describe the present disclosure in more detail, and the scope of the present disclosure is not limited to the following examples.

### [Examples]

### Example 1

Isopropyl alcohol was produced and purified according to a process system as shown in FIGS. 1 and 2, and at this time, a purified isopropyl alcohol (IPA) stream recovered in the purification process was passed through the arsenic removal device 400 as shown in FIG. 3 to remove arsenic in IPA.

Specifically, as shown in FIG. 3, 10 m³/h of an IPA stream having an arsenic (As (V)) concentration of 100 ppt was passed through the arsenic removal device 400 having a volume of 10 m³ having an inner structure in which a first filtration layer 11, an adsorbent layer 20 including an arsenic adsorbent based on iron hydroxide, and a second filtration layer 12 were laminated sequentially under room temperature and normal pressure conditions. That is, when the empty bed contact time (EBCT) was 1 hour, as a result of measuring an arsenic concentration of IPA after passing through the arsenic removal device, the arsenic removal rate was 45 wt%.

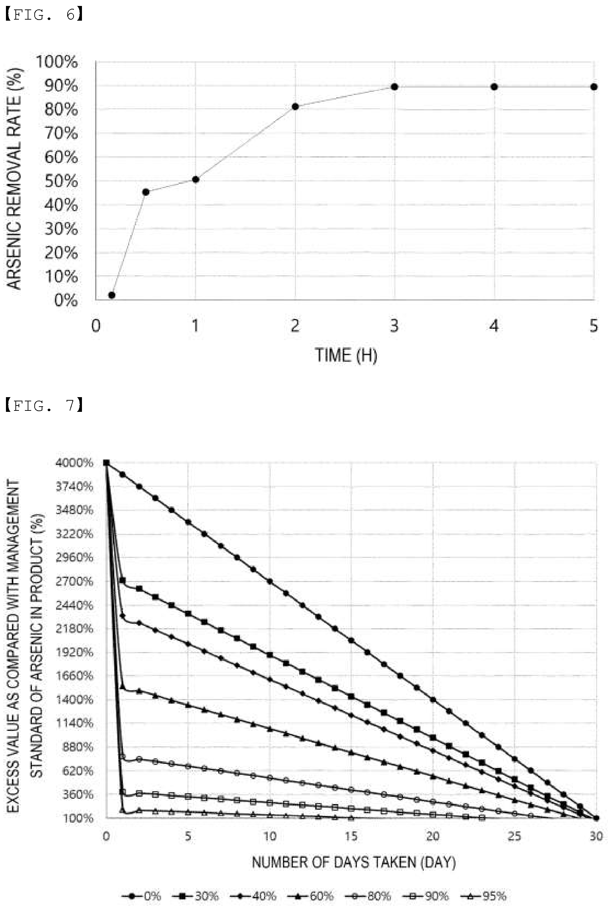

In this connection, FIG. 6 is a graph showing the arsenic removal rate depending on the empty bed contact time (EBCT) of IPA passing through the arsenic removal device.

Referring to FIG. 6, it was found that when the empty bed contact time (EBCT) of IPA passing through the arsenic removal device having a volume of 10 m³ was 30 minutes, the arsenic removal rate was about 45 wt%, when EBCT was 1 hour, the arsenic removal rate was about 50 wt%, and when EBCT was 2 hours or more, about 80 wt% or more of arsenic was removed. For reference, in FIG. 6, the reason that when EBCT was 3-5 hours, the arsenic removal rate was shown to be all 90% was that it was beyond the measurement limit.

Referring to the fact, assuming a process in which the arsenic removal device having the same specification was used, and the volume flow rate of purified IPA was fixed to 10 m³/h, when the total amount of the IPA stream of 10 m³/h was added to the arsenic removal device, the arsenic removal rate was 50 wt%, and the IPA stream having no arsenic of 5 m³/g was able to be obtained.

However, when the IPA stream was partly branched, and the IPA stream of 5 m³/h was not passed through the arsenic removal device and only the rest of the IPA stream of 5 m³/h was added to the arsenic removal device, 80 wt% of arsenic was removed only from the rest of the IPA stream of 5 m³/h and arsenic was not removed from the part of the IPA stream of 5 m³/h, and thus, only the IPA stream from which a total of 4 m³/h of arsenic was removed was able to be obtained. Therefore, even in the case of using the same device, the arsenic removal efficiency may vary depending on the flow rate of the IPA stream added to the device.

### [Experimental Examples]

When arsenic was introduced to the process due to the catalyst exchange in the IPA process, the arsenic concentration in a product began to be detected at a numerical value about 40 times higher than the management standard, and the arsenic concentration tended to decrease over time. Since the flow rate of the circulation water was 8 to 9 times higher than the flow rate of the purified IPA stream due to the IPA preparation process characteristics, and circulated and recycled in the process, most of the arsenic once concentrated in the process was concentrated in the process water, only a part was included in IPA and eluted, and thus, when arsenic at a high concentration was added to the process once, it stayed in the process for a long time.

When the arsenic removal process is introduced to the purified IPA stream having a relatively low flow rate, the arsenic removal rate may be increased while decreasing a device size and an absorbent use amount. Thus, in the present experimental example, in order to confirm the effect of remove arsenic in the produce depending on whether the arsenic removal process according to the present disclosure was introduced, arsenic was added to the IPA preparation process so that 40 times or more arsenic was detected as compared with the management standard and the change of the arsenic concentration in the IPA product over time was confirmed, and the results are shown in Table 1 and FIG. 7.

In the following Table 1, Comparative Experimental Example 1 did not perform the arsenic removal process according to the present disclosure, and Experimental Examples 1 to 6 performed the arsenic removal process according to the present disclosure as in Example 1. Specifically, in Experimental Examples 1 to 6, the arsenic removal device having the same size was used, but the IPA flow rate was adjusted so that it had different empty bed contact time as shown in Table 1 and the arsenic removal experiment was performed.

In addition, the "number of days taken" in the following Table 1 and FIG. 7 was measurement of a period from the time when arsenic was added until the arsenic concentration in the IPA product was normalized, that is, the number of days it took for the arsenic concentration in the product to reach 100% from 4,000% as compared with the management standard for each arsenic removal rate (wt%) of the IPA. In addition, the "number of days shortened" was a calculation of a shortened degree of the days taken in arsenic concentration normalization of Experimental Examples 1 to 6, based on the number of days taken in Comparative Experimental Example 1.

**[Table 1]**

| | Empty bed contact time (h) | Arsenic removal rate (%) | Number of days taken (day) | Number of days shortened (day) |
|---|---|---|---|---|
| Comparative Experimental Example 1 | - | 0 | 30 | - |
| Experimental Example 1 | 0.65 | 30 | 30 | 0 |
| Experimental Example 2 | 0.82 | 40 | 30 | 0 |
| Experimental Example 3 | 1.5 | 60 | 29 | 1 |
| Experimental Example 4 | 2 | 80 | 27 | 3 |
| Experimental Example 5 | 3 | 90 | 24 | 6 |
| Experimental Example 6 | 5 (>3h) | 95 | 15 | 15 |

Referring to Table 1 and FIG. 7, in Comparative Experimental Example 1 in which the arsenic removal process according to the present disclosure was not performed, the arsenic concentration was lowered to 100% from 4,000% over about a month, as compared with the management standard. This means that IPA produced for 30 days did not satisfy the management standard and was inappropriate for IPA used in a semiconductor process.

Meanwhile, in Experimental Examples 3 to 6 to which the arsenic removal process according to the present disclosure was introduced were able to shorten the period from the time when the arsenic was added until arsenic concentration in the product was normalized, by removing arsenic in the process water. However, in Experimental Examples 1 and 2 in which the arsenic removal rate in the product was 40% or less, the number of days taken was not shortened. In addition, as the arsenic removal rate in the IPA was higher, the number of days it took for the arsenic concentration to be normalized was further shortened, and a high-quality product production amount was able to be secured by the shortened number of days, which was advantageous from an economic perspective.

Furthermore, under the process conditions in which the flow rate of produced IPA was constant, the arsenic removal rate may be increased as the size of the arsenic removal device was larger, and the adsorbent use amount was larger. Considering these facts, it is considered that the process needs to be appropriately designed after comparing costs incurred when increasing the volume of the arsenic removal device for improving the arsenic removal rate and profits which may be obtained from the product production amount increased depending on the improved arsenic removal rate.

Hereinabove, the exemplary embodiments of the present disclosure have been described, but the present disclosure is not limited thereto, and those with ordinary skill in the art will understand that various changes and modification are possible within the range which is not out of the concept and the scope of the claims described later.

### [Reference signs list]

- 100:: Reactor
- 201:: Absorption tower
- 202:: Gas purification part
- 301:: Organic material removal tower
- 302:: Water removal tower
- 303:: IPA separation part
- 400:: Arsenic removal device
- 11, 12:: Filtration layer
- 20:: Adsorbent layer

## Claims

1. A method for preparing isopropyl alcohol, the method comprising:
(S1) supplying a propylene monomer and reaction water to a reactor as a feed stream to produce a gaseous reaction product including isopropyl alcohol;
(S2) purifying isopropyl alcohol from the gaseous reaction product and recovering process water; and
(S3) passing the purified isopropyl alcohol through an arsenic removal device including a first filtration layer, an adsorbent layer, and a second filtration layer,
wherein (S3) includes passing the purified isopropyl alcohol through the first filtration layer, the adsorbent layer, and the second filtration layer inside the arsenic removal device sequentially.

2. The method for preparing isopropyl alcohol of claim 1, wherein the first filtration layer, the adsorbent layer, and the second filtration layer are laminated sequentially in the arsenic removal device.

3. The method for preparing isopropyl alcohol of claim 1, wherein the adsorbent layer is divided into first to nth areas, and the first to nth areas are filled with first to nth adsorbents, respectively.

4. The method for preparing isopropyl alcohol of claim 3, wherein when the purified isopropyl alcohol passes through the adsorbent layer, the purified isopropyl alcohol is supplied to one side of the first area, passes from the first area to the nth area sequentially, and then is discharged in one direction selected from one side and the other side of the nth area.

5. The method for preparing isopropyl alcohol of claim 3, wherein n is an integer of 2 to 5.

6. The method for preparing isopropyl alcohol of claim 1, wherein the adsorbent layer includes one or more arsenic adsorbents selected from iron hydroxide, activated alumina, titanium oxide, α-iron oxide, γ-iron oxide, and an ion exchange resin.

7. The method for preparing isopropyl alcohol of claim 1, wherein the first filtration layer and the second filtration layer independently of each other include one or more selected from activated carbon, sand, zeolite, silica gel, and mesh net.

8. The method for preparing isopropyl alcohol of claim 1, wherein an empty bed contact time of the arsenic removal device is 10 minutes to 5 hours.

9. The method for preparing isopropyl alcohol of claim 1, wherein a temperature of isopropyl alcohol passing through the arsenic removal device is 30°C to 120°C.

10. The method for preparing isopropyl alcohol of claim 1, wherein an arsenic removal rate after passing through the arsenic removal device is 30 wt% or more, based on 100 wt% of an arsenic content included in the isopropyl alcohol before passing through the arsenic removal device.

11. The method for preparing isopropyl alcohol of claim 1, wherein the gaseous reaction product includes isopropyl alcohol, unreacted propylene, unreacted water, and by-products.

12. The method for preparing isopropyl alcohol of claim 1, wherein (S2) includes:
(i) supplying the gaseous reaction product to an absorption tower and bringing the gaseous reaction product into contact with washing water to obtain an aqueous solution including isopropyl alcohol,
(ii) supplying the aqueous solution including isopropyl alcohol to a gas purification part and separating an upper stream including low-boiling point components and a lower stream including isopropyl alcohol, water, and by-products,
(iii) supplying the lower stream of the gas purification part to an organic material removal tower and bringing the stream into contact with washing water to separate a liquid phase including isopropyl alcohol and water and a liquid phase including an organic material, and
(iv) supplying the liquid phase including isopropyl alcohol and water to a water removal tower to separate an upper stream including isopropyl alcohol and a lower stream of water, purifying isopropyl alcohol from the upper stream, and recovering the lower stream as the process water.

13. The method for preparing isopropyl alcohol of claim 12, wherein the washing water used in the absorption tower and the organic material removal tower is the process water recovered in (S2).
